(19) Europäisches Patentamt

European Patent Office

Office européen des brevets

(11) Veröffentlichungsnummer: **0 533 003 B1**

(12) **EUROPÄISCHE PATENTSCHRIFT**

(45) Veröffentlichungstag der Patentschrift: 06.12.95

(51) Int. Cl.6: **C07C 17/00**, C07C 17/20, C07C 19/08, B01J 27/053, B01J 27/06

(21) Anmeldenummer: 92115231.0

(22) Anmeldetag: 05.09.92

(54) Verfahren zur Herstellung von Fluor enthaltenden Ethanderivaten.

(30) Priorität: 14.09.91 DE 4130696

(43) Veröffentlichungstag der Anmeldung:
24.03.93 Patentblatt 93/12

(45) Bekanntmachung des Hinweises auf die Patenterteilung:
06.12.95 Patentblatt 95/49

(84) Benannte Vertragsstaaten:
AT BE CH DE DK ES FR GB GR IE IT LI LU NL PT

(56) Entgegenhaltungen:
EP-A- 0 297 947
EP-A- 0 451 746
WO-A-89/12617

JOURNAL OF FLUORINE CHEMISTRY Bd. 47, Nr. 2, Mai 1990, LAUSANNE CH Seiten 317 - 332 WALTER C. CICHA ET AL. 'Niobium(V) and Tantalum(V) Fluoride Derivatives of Fluorosulfuric and Trifluoromethylsulfuric Acids'

(73) Patentinhaber: Solvay Fluor und Derivate GmbH
Hans-Böckler-Allee 20
D-30173 Hannover (DE)

(72) Erfinder: Eicher, Johannes
Moorkamp 7
W-3008 Garbsen 5 (DE)
Erfinder: Fazniewscy, Karl-Heinz
Im Gesenk 8
W-3160 Lehrte (DE)
Erfinder: Rudolph, Werner
Oderstrasse 38
W-3000 Hannover 71 (DE)
Erfinder: Swidersky, Hans-Walter
Zeppelinstrasse 5
W-3000 Hannover 1 (DE)

(74) Vertreter: Lauer, Dieter, Dr.
Solvay Pharma Deutschland GmbH
Hans-Böckler-Allee 20
D-30173 Hannover (DE)

**Beschreibung**

Die Erfindung bezieht sich auf ein Verfahren zur Herstellung von Fluor enthaltenden Ethanderivaten der allgemeinen Formel $F_kCH_nCl_{3-k-n}CZ^1Z^2F$, worin k, n, $Z^1$ und $Z^2$ die unten beschriebene Bedeutung besitzen.

Es besteht ein zunehmender Bedarf an umweltverträglichen Halogenkohlenwasserstoffen. Als solche haben sich beispielsweise jene Fluor enthaltenden Ethanderivate erwiesen, welche mindestens ein Wasserstoffatom aufweisen, beispielsweise $CF_3CH_3$ (R143a), $CF_3CH_2Cl$ (R133a) und besonders $CF_3CHCl_2$ (R123) sowie $CF_3CH_2F$ (R134a). Interessant sind aber auch die entsprechenden 1-Fluor- oder 1.1.-Difluorverbindungen, beispielsweise die als umweltverträglich geltenden Verbindungen $CFCl_2CHCl_2$ oder $CF_2ClCHCl_2$, die als Kältemittel, Lösungsmittel oder Treibmittel einsetzbar sind.

Industriell werden solche Verbindungen durch katalysierten Halogen-Fluor-Austausch, insbesondere durch Chlor-Fluor-Austausch, aus entsprechend halogenierten Derivaten hergestellt. Die hierbei verwendeten halogenierten Ausgangsverbindungen sind gegenüber einem Halogen-Fluor-Austauch jedoch sehr reaktionsträge.

Besonders zur Herstellung höherfluorierter Verbindungen sind drastische Verfahrensbedingungen notwendig. Trotz solcher drastischer Bedingungen, beispielsweise das Arbeiten in der Gasphase, sind die Umsätze gewöhnlich gering. Weiterer Nachteil bekannter Verfahren ist, daß die verwendeten, oft sehr teuren Katalysatoren keine zufriedenstellende Lebensdauer aufweisen.

Aufgabe der vorliegenden Erfindung ist es, ein Verfahren zur Herstellung von Fluor enthaltenden Ethanderivaten zur Verfügung zu stellen, das technisch einfach durchzuführen ist und einen hohen Umsatz aufweist.

Diese Aufgabe wird durch das Verfahren der vorliegenden Erfindung gelöst.

Das erfindungsgemäße Verfahren umfaßt die Herstellung von Fluor enthaltenden Ethanderivaten der allgemeinen Formel (I)

$$F_kH_nCl_{3-k-n}C\text{-}CZ^1Z^2F \qquad (I)$$

worin $Z^1$ und $Z^2$ gleich oder verschieden sein können und Wasserstoff, Fluor, Chlor oder Brom bedeuten und k 0, 1 oder 2 und n 1, 2 oder 3 bedeuten
durch Umsetzung eines Alkens oder halogenhaltigen Alkans als Ausgangsverbindung mit Fluorwasserstoff in flüssiger Phase bei einer Temperatur zwischen 0 und 250 °C, wobei die Umsetzung durch eine katalytisch aktive Zubereitung enthaltend eine Tantal-Fluorsulfonat-Verbindung und/oder eine Niob-Fluorsulfonat-Verbindung, die im wesentlichen frei von Tantal- und/oder Niobpentahalogenid, vorzugsweise im wesentlichen frei von Tantal- und/oder Niobpentachlorid und Tantal- und/oder Niobpentabromid ist, katalysiert wird und der Fluorwasserstoff mindestens in äquimolarer Menge, bezogen auf die Ausgangsverbindung, eingesetzt wird, das Mol-Verhältnis von Ausgangsverbindung zu katalytisch aktiver Verbindung zwischen etwa 10:1 bis 1:100 beträgt, wobei man

a) als Alken eine Verbindung der allgemeinen Formel (II) verwendet

$$F_kH_mCl_{2-k-m}C = CX^1X^2 \qquad (II)$$

worin $X^1$ und $X^2$ gleich oder verschieden sein können und Wasserstoff, Fluor, Chlor oder Brom bedeuten, k 0, 1 oder 2 und m 0, 1 oder 2 bedeutet, oder

b) als halogenhaltiges Alkan eine Verbindung der allgemeinen Formel (III) verwendet

$$F_kH_nCl_{3-k-n}C\text{-}CY^1Y^2Y^3 \qquad (III)$$

worin k und n die obige Bedeutung besitzt, $Y^1$, $Y^2$ gleich oder verschieden sein können und Wasserstoff, Fluor, Chlor oder Brom und $Y^3$ Chlor oder Brom bedeuten.

Im Rahmen der vorliegenden Erfindung ist die Summe aus k und n 1, 2 oder 3 und die Summe aus k und m 0, 1 oder 2.

Bevorzugt steht in den Ausgangsverbindungen der Formel II und III k für 0 und $X^1$, $X^2$, $Y^1$ und $Y^2$ für Fluor, Chlor oder Brom.

Das Verfahren kann bei Drücken zwischen 1 und 100 bar (abs.) und Temperaturen von 0 bis 250 °C, vorzugsweise 50 bis 250 °C betrieben werden. Hierbei werden Druck und Temperatur so gewählt, daß die Umsetzung in der flüssigen Phase abläuft.

Als katalytisch aktive Zubereitung verwendet man bevorzugt eine Tantal-Halogenid-Fluorsulfonat-Verbindung oder eine Niob-Halogenid-Fluorsulfonat-Verbindung der Formel $MX_n (FSO_3)_{5-n}$, worin X Halogenid, M Niob oder Tantal bedeutet und n = 0 bis 4 ist. "Halogenid" bedeutet hier vorzugsweise Chlorid oder Fluorid, insbesondere Chlorid, und n bedeutet bevorzugt 1 bis 4, insbesondere 2 bis 4.

Die Herstellung solcher Verbindungen ist bekannt.

So beschreiben E. Hayek, J. Puschmann und A. Czaloun in Monatsh. Chem. 85 (1954), Seiten 359 bis 363 die Herstellung von $TaCl_3 (FSO_3)_2$ aus Tantalpentachlorid und Fluorsulfonsäure und Abdampfen überschüssiger Fluorsulfonsäure. Die Verbindung läßt sich auch aus Tantalpentachlorid und Fluorsulfonsäurederivaten, beispielsweise Fluorsulfonsäureethylester herstellen. Reaktionsprodukte aus Niobpentachlorid und Fluorsulfonsäure sind viskos oder gallertartig. Nach Ansicht der Autoren H.C. Clark und H.J. Emeleus in J. Chem. Soc. 1958, Seiten 190 bis 195, insbesondere auf Seite 193, handelt es sich um $NbCl_3 (FSO_3)_2$.

Die Autoren H.C. Clark und H.J. Emeleus beschreiben an gleicher Stelle auch die Herstellung von $TaF_3 (FSO_3)_2$ und $NbF_3 (FSO_3)_2$ aus Tantalpentafluorid bzw. Niobpentafluorid und Schwefeltrioxid bei Raumtemperatur.

Die Autoren W.V. Cicha und F. Aubke beschreiben in J. Fluorine Chem. 47 (1990), Seiten 317 bis 332 die Herstellung von weiteren Tantal- und Niob-Fluorid-Fluorsulfonat-Verbindungen.

$Ta(FSO_3)_5$ und $Nb(FSO_3)_5$ können durch Oxidation des jeweiligen Metallpulvers mit bis-(Fluorosulfuryl)-peroxid in Fluorsulfonsäure hergestellt werden. Bei der Umsetzung von Niobmetallpulver entsteht als Nebenprodukt auch $NbF_2 (FSO_3)_3$.

$TaF_4 (FSO_3)$ läßt sich beispielsweise durch Ligandenaustauschreaktion zwischen $Ta(FSO_3)_5$ und 4 Moläquivalenten Tantalpentafluorid herstellen.

Wie gesagt, verwendet man vorzugsweise eine Tantal- oder Niob-Halogenid-Fluorsulfonat-Verbindung.

Gemäß einer besonders bevorzugten Ausführungsform ist die katalytisch aktive Verbindung eine Tantal-Chlorid-Fluorsufonat-Verbindung ober eine Niob-Chlorid-Fluorsulfonat-Verbindung, die durch die Umsetzung von Tantalpentachlorid oder Niobpentachlorid mit Fluorsulfonsäure im Mol-Verhältnis von 1:1 bis 1:4 erhalten wurde. Anstelle der Fluorsulfonsäure kann man auch Fluorsulfonsäurederivate verwenden, die mit Tantalpentachlorid oder Niobpentachlorid unter Substition von Chlorid durch Fluorsulfonat reagieren, beispielsweise Fluorsulfonsäureester.

Gemäß einer anderen besonders bevorzugten Ausführungsform verwendet man als katalytisch aktive Verbindung eine Tantal-Fluorid-Fluorsulfonat-Verbindung oder eine Niob-Fluorid-Fluorsulfonat-Verbindung, die durch Umsetzung von Tantalpentafluorid oder Niobpentafluorid mit Schwefeltrioxid erhalten wurde. Alternativ verwendet man entsprechende Verbindungen, die durch Umsetzung von Tantalpentachlorid oder Niobpentachlorid mit Fluorsulfonsäure oder einem Fluorsulfonsäurederivat, welches mit Tantalpentachlorid oder Niobpentachlorid unter Substition von Chlorid durch Fluorsulfonat reagiert, beispielsweise einem Fluorsulfonsäureester, im Mol-Verhältnis von 1:1 bis 1:4 und anschließendem Chlor-Fluor-Austausch erhalten wurden.

Wie gesagt, wird das erfindungsgemäße Verfahren durch eine katalytisch aktive Zubereitung katalysiert, die im wesentlichen frei von Tantal- bzw. Niobpentahalogenid sein soll. Es sind also allenfalls noch geringe, unerwünschte Mengen an solchen Pentahalogeniden in der Zubereitung vorhanden.

Geht der Fachmann von einer Zubereitung aus, die durch Umsetzung des betreffenden Niob- oder Tantalmetallpulvers mit bis-(Fluorosulfuryl)peroxid in Fluorsulfonsäure hergestellt wurde, ist die Zubereitung naturgemäß frei von Pentahalogenid.

Geht der Fachmann aus von Zubereitungen, die durch Umsetzung von Tantal- oder Niobpentahalogenid und Fluorsulfonsäure, Fluorsulfonsäurederivaten oder Schwefeltrioxid hergestellt wurden, so setzt er Fluorsulfonsäure und das Pentahalogenid mindestens im Verhältnis von 1:1 ein. Die Umsetzung zwischen Alken bzw. Alkan und Fluorwasserstoff wird erst dann durchgeführt, wenn Tantal- bzw. Niob-Pentahalogenid und Fluorsulfonsäure bzw. ein Derivat der Fluorsulfonsäure oder Schwefeltrioxid unter Bildung der entsprechenden Tantal- bzw. Niob-Halogenid-Fluorsulfonat-Verbindung reagiert haben und die Zubereitung im wesentlichen frei von Pentahalogenid ist. Dies schließt nicht aus, daß entweder das zu (hydro)fluorierende Alken bzw. Alkan oder Fluorwasserstoff vor oder während der Umsetzung von Niob- oder Tantalpentahalogenid zu Niob- bzw. Tantal-Halogenid-Fluorsulfonat-Verbindung in dieser Reaktionsmischung vorhanden sein kann. Beispielsweise kann man Niob- oder Tantalpentachlorid und Fluorsulfonsäure in Anwesenheit des zu (hydro)fluorierenden Alkens oder Alkans miteinander umsetzen. Die organische Verbindung wirkt dann als Verdünnungsmittel. Der für die Hydrofluorierung oder den Chlor-Fluor-Austausch am Alken bzw. Alkan benötigte Fluorwasserstoff wird in diesem Fall erst dann eingeleitet, wenn das Pentahalogenid zur Bildung der Halogenid-Fluorsulfonat-Verbindung im wesentlichen verbraucht ist. Bereits bei Beginn der Umsetzung zwischen Alken/Alkan und Fluorwasserstoff soll die Zubereitung im wesentlichen frei von Tantal- bzw. Niob-Pentahalogenid sein.

Die beim erfindungsgemäßen Verfahren erhaltenen Ethanderivate unterscheiden sich von den Ausgangsverbindungen dadurch, daß sie mindestens ein Fluoratom mehr aufweisen. Für jedes auf diese Weise in das Substratmolekül eingeführte Fluoratom setzt man zweckmäßigerweise Fluorwasserstoff in einer Menge ein, die mindestens der stöchiometrisch notwendigen Menge enspricht. Die zur HF-Anlagerung bzw. zum Halogen-Fluor-Austausch eingesetzte Menge an Fluorwasserstoff kann auch größer sein. Sie kann beispielsweise das bis zu fünfzehnfache und mehr der stöchiometrisch notwendigen Menge betragen.

Bei der Herstellung trifluormethylgruppenhaltiger Derivate aus ungesättigten Verbindungen werden gute Ergebnisse bereits dann erzielt, wenn die eingesetzte Menge an Fluorwasserstoff dem ein- bis zehnfachen der stöchiometrisch notwendigen Menge entspricht.

Die einzusetzende Menge an Fluorwasserstoff kann über diese Menge, die zur Fluorwasserstoffanlagerung und/oder zum Chlor-Fluor-Austausch am Alken oder Alkan benötigt wird, hinausgehen. Wenn man nämlich Ta- oder Nb-Halogenid-Fluorsulfonat-Verbindungen als Katalysatorbestandteil einsetzt, welche Chlorid oder Bromid aufweisen, liegen diese Metallhalogenide möglicherweise durch Austausch von Chlor oder Brom gegen Fluor in Form von mehr oder weniger Fluor enthaltenden Metallhalogenid-Fluorsulfonaten in der Reaktionsmischung vor.

Die weiter oben gemachte Angabe zur einzusetzenden Menge an Fluorwasserstoff ist also in dem Sinn zu verstehen, daß man für jedes in das Substratmolekül eingeführte Fluoratom zweckmäßigerweise Fluorwasserstoff in einer Menge einsetzt, die mindestens der stöchiometrisch notwendigen Menge entspricht, und zusätzlich noch soviel Fluorwasserstoff, wie für einen etwaigen Halogen-Fluor-Austausch des Halogenids im Metall-halogenid-fluorsulfonat benötigt wird. Sinngemäß sind auch die weiter unten gemachten Angaben, die sich mit der Stöchiometrie des für die Fluorwasserstoffaddition und/oder den Halogen-Fluor-Austausch beim Alken oder Alkan einzusetzenden Fluorwasserstoffs befassen, aufzufassen. Der Einfachheit halber wird in diesen Angaben nicht jedesmal ausdrücklich erwähnt, daß zusätzlicher Fluorwasserstoff für den etwaigen Halogen-Fluor-Austausch des Halogenids notwendig sein kann.

Um abzuschätzen, welche Menge zusätzlich an Fluorwasserstoff für diesen Halogen-Fluor-Austausch notwendig wird, kann der Fachmann das einzusetzende Metallhalogenid-Fluorsulfonat vorab mit Fluorwasserstoff umsetzen. Die Menge an verbrauchtem Fluorwasserstoff und/oder die Menge an gebildetem Halogenwasserstoff ermöglicht es, zu berechnen, welche Menge an Fluorwasserstoff zusätzlich zu der für die Umsetzung des eingesetzten Halogenkohlenwasserstoffs hinaus benötigt wird.

Ganz besonders einfach und zweckmäßig ist folgende Vorgehensweise: Das Metallhalogenid, beispielsweise Niobpentachlorid oder -bromid oder Tantalpentachlorid oder -bromid wird im Fluorierungsreaktor, ggf. in Anwesenheit eines Teils oder der gesamten zu fluorierenden organischen Ausgangsverbindung, vorgelegt und mit Fluorsulfonsäure versetzt, bis sich kein Chlorwasserstoff bzw. Bromwasserstoff mehr entwickelt. Dann leitet man ggf. den Rest des zu fluorierenden Halogenkohlenwasserstoffs und den zur Fluorierung benötigten Fluorwasserstoff in das Katalysatorgemisch ein.

Es ist zweckmäßig, zur Herstellung niedrigfluorierter Verbindungen, beispielsweise von $CHCl_2CCl_2F$ aus $CHCl_2CCl_3$ oder $CCl_2=CCl_2$, von $CH_2ClCCl_2F$ aus $CH_2ClCCl_3$ oder $CHCl=CCl_2$ oder von $CH_3CCl_2F$ aus $CH_3CCl_3$ oder $CH_2=CCl_2$, im unteren Temperaturbereich, beispielsweise zwischen 50 und 150 °C zu arbeiten. Der Umsetzungsgrad kann analytisch verfolgt werden, beispielsweise durch Probenentnahme und Untersuchung durch Gaschromatographie.

Vorteilhaft ist hier der hohe Umsetzungsgrad des erfindungsgemäßen Verfahrens.

In der folgenden Tabelle 1 sind, unter Angabe von einsetzbaren Ausgangsverbindungen und der stöchiometrisch notwendigen Menge an Fluorwasserstoff, einige Beispiele für erfindungsgemäß herstellbare Fluor enthaltende Ethanderivate angegeben.

## Tabelle 1

$$CCl_2 = CCl_2 + HF \rightarrow CHCl_2 CCl_2 F$$

$$CCl_2 = CCl_2 + 3HF \rightarrow CHCl_2 CF_3$$

$$CHCl_2 CCl_3 + 3HF \rightarrow CHCl_2 CF_3$$

$$CHCl_2 CCl_2 F + 2HF \rightarrow CHCl_2 CF_3$$

$$CHCl_2 CClF_2 + HF \rightarrow CHCl_2 CF_3$$

$$CHCl = CCl_2 + 3HF \rightarrow CH_2 ClCF_3$$

$$CH_2 ClCCl_3 + 3HF \rightarrow CH_2 ClCF_3$$

$$CH_2 = CCl_2 + 3HF \rightarrow CH_3 CF_3$$

$$CH_3 CCl_3 + 3HF \rightarrow CH_3 CF_3$$

$$CFH = CF_2 + HF \rightarrow CH_2 FCF_3$$

Die Vorteile des erfindungsgemäßen Verfahrens zeigen sich ganz besonders bei der Herstellung höherfluorierter Produkte. Bei der Herstellung höherfluorierter Produkte, beispielsweise der Herstellung von $CHCl_2 CF_3$ aus $CHCl_2 CCl_3$, $CHCl_2 CCl_2 F$, $CHCl_2 CClF_2$, $CCl_2 = CCl_2$ oder deren Gemischen, zur Herstellung von $CH_2 ClCF_3$ aus $CH_2 ClCCl_3$, $CH_2 ClCCl_2 F$, $CH_2 ClCClF_2$, $CHCl = CCl_2$ oder deren Gemischen oder zur Herstellung von $CH_3 CF_3$ aus $CH_3 CCl_3$, $CH_3 CCl_2 F$, $CH_3 CClF_2$, $CH_2 = CCl_2$ oder deren Gemischen, arbeitet man zweckmäßigerweise im höheren Temperaturbereich, beispielsweise zwischen 70 und 220 °C und 10 bis 50 bar. Auch hier kann der Umsetzungsgrad durch Analyse von gezogenen Proben der Reaktionsmischung ermittelt werden.

Besonders vorteilhaft ist das erfindungsgemäße Verfahren deshalb zur Herstellung höherfluorierter Verbindungen, insbesondere von trifluormethylgruppenhaltigen Ethanderivaten der allgemeinen Formel $F_k H_n Cl_{3-k-n} C\text{-}CF_3$ (Ia), in welchen k 0, 1 oder 2 und n 1, 2 oder 3 bedeutet.

Diese bevorzugte Ausführungsform des erfindungsgemäßen Verfahrens ist dadurch gekennzeichnet, daß man zur Herstellung von Fluor enthaltenden Ethanderivaten der allgemeinen Formel (Ia)

$$F_k H_n Cl_{3-k-n} C\text{-}CF_3 \qquad (Ia)$$

worin k 0, 1 oder 2 und n 1, 2 oder 3 bedeutet, bei einer Temperatur von 50 bis 250 °C und einem Druck von 1 bis 100 bar (abs.)

a) ein halogenhaltiges Alken der allgemeinen Formel

$$F_k H_m Cl_{2-k-m} C = CX^1 X^2 \qquad (II),$$

worin k und m die oben angegebene Bedeutung besitzen, $X^1$ und $X^2$ Fluor, Chlor oder Brom bedeuten, mit Fluorwasserstoff umsetzt, wobei der Fluorwasserstoff mindestens in der stöchiometrisch zur Fluorwasserstoffanlagerung und zum Halogen-Fluor-Austausch am Alken notwendigen Menge eingesetzt wird oder

b) ein halogenhaltiges Alkan der allgemeinen Formel

$$F_k H_n Cl_{3-k-n} C\text{-}CY^1 Y^2 Y^3 \qquad (III)$$

worin k und n die oben angegebene Bedeutung besitzen und $Y^1$ und $Y^2$ Fluor, Chlor oder Brom und $Y^3$ Chlor oder Brom bedeuten, mit Fluorwasserstoff umsetzt, wobei der Fluorwasserstoff mindestens in der stöchiometrisch zum Halogen-Fluor-Austausch am Alkan notwendigen Menge eingesetzt wird.

Besonders augenfällig werden die Vorteile des erfindungsgemäßen Verfahrens, wenn man von den entsprechenden halogenierten Alkenen als Ausgangsverbindung ausgeht. Der Umsatzgrad bei Anwendung dieser eigentlich sehr reaktionsträgen Verbindungen ist sehr hoch. Die bei anderen Verfahren beobachtete Bildung von Polymerisaten sowie von Verbindungen, die nicht durch Anlagerung von Fluorwasserstoff, sondern durch Anlagerung von Halogen entstanden sind, wird beim erfindungsgemäßen Verfahren allenfalls in verschwindend geringer Menge beobachtet.

Gemäß einer bevorzugten Variante des erfindungsgemäßen Verfahrens setzt man halogenierte Alkene als Ausgangsverbindungen ein. In einem ersten Reaktionsschritt bilden sich zunächst Fluorwasserstoffadditions-Verbindungen. Bezweckt man die Herstellung derartiger niedrig fluorierter Verbindungen, die ja wertvolle Eigenschaften aufweisen, beispielsweise als Lösungsmittel, isoliert man diese Verbindungen aus der Reaktionsmischung. Bezweckt man die Herstellung höherfluorierter Produkte, insbesondere von trifluormethylgruppenhaltigen Ethanderivaten, kann man diese Verbindungen isolieren und weiter fluorieren. Zweckmäßigerweise isoliert man diese intermediär in einem ersten Reaktionsschritt entstehenden Fluorwasserstoffadditions-Verbindungen nicht, sondern setzt diese Verbindungen in situ mit weiterem Fluorwasserstoff zu den gewünschten höher fluorierten Produkten um.

Man kann auch von mindestens teilweise fluorierten Alkenen ausgehen. So kann gemäß einer Ausführungsform des erfindungsgemäßen Verfahrens $CH_2FCF_3$ hergestellt werden, indem Trifluorethylen mit Fluorwasserstoff in Anwesenheit des Metallhalogenid-fluorsulfonats umgesetzt wird. Erstaunlicherweise wird eine Polymerisation des Alkens nicht beobachtet.

Hervorragend geeignet ist das erfindungsgemäße Verfahren zur Herstellung von $CHCl_2CF_3$ (R123). Diese ganz besonders bevorzugte Ausführungsform des erfindungsgemäßen Verfahrens ist dadurch gekennzeichnet, daß man zur Herstellung von $CHCl_2CF_3$

a) $CCl_2 = CCl_2$ mit Fluorwasserstoff umsetzt, wobei Fluorwasserstoff mindestens in der stöchiometrisch zur Fluorwasserstoff-Anlagerung und zum Chlor-Fluor-Austausch am Alken notwendigen Menge eingesetzt wird, oder

b) $CHCl_2CCl_3$, $CHCl_2CFCl_2$, $CHCl_2CF_2Cl$ oder deren Gemische mit Fluorwasserstoff umsetzt, wobei Fluorwasserstoff mindestens in der stöchiometrisch zum Chlor-Fluor-Austausch am Alkan notwendig Menge eingesetzt wird.

Ganz besonders bevorzugt ist die Herstellung von $CHCl_2CF_3$ aus $CCl_2 = CCl_2$ nach Variante a). Das Mol-Verhältnis von $CCl_2 = CCl_2$ und Fluorwasserstoff liegt vorteilhaft zwischen 1:3 und 1:100.

Das Mol-Verhältnis von Ausgangsverbindung zum Metall-fluorsulfonat-Verbindung liegt, wie weiter oben angeführt, zwischen 10:1 und 1:100.

Bevorzugt liegt das Mol-Verhältnis von Ausgangsverbindung zum Metall-fluorsulfonat-Verbindung zwischen 10:1 und 1:10, besonders bevorzugt zwischen 2:1 und 1:5.

Eine ganz besonders bevorzugte Ausführungsform des Verfahren ist dadurch gekennzeichnet, daß man zur Herstellung von $CHCl_2CF_3$ ausgeht von $CCl_2 = CCl_2$, dieses mit der stöchiometrisch zur Fluorwasserstoffanlagerung und zum Chlor-Fluor-Austausch am Alken mindestens notwendigen Menge an Fluorwasserstoff umsetzt, wobei man als katalytisch aktive Zubereitung das Reaktionsprodukt der Umsetzung von Niobpentahalogenid, Tantalpentahalogenid oder Gemische dieser Verbindungen mit Fluorsulfonsäure im Molverhältnis von 1:1 bis 1:3 einsetzt.

Feuchtigkeit wirkt sich im erfindungsgemäßen Verfahren störend aus. Die Umsetzung wird daher zweckmäßigerweise unter Bedingungen durchgeführt, die verhindern, daß eine schädliche Menge an Wasser in die Reaktionsmischung gelangen kann. Man verwendet im wesentlichen wasserfreien Fluorwasserstoff. Je nach eingesetzter Menge an Fluorwasserstoff kann es empfehlenswert sein, den handelsüblichen Fluorwasserstoff vor seiner Verwendung zu trocknen. Weiterhin ist es empfehlenswert, die verwendete Apparatur im möglichst trockenen Zustand zu halten. Hierzu kann man Leitungen, Reaktionsgefäße, Einrichtungen zur Produktaufarbeitung und -Aufbewahrung mit trockenen Gasen spülen, beispielsweise mit trockener Luft oder trockenem Stickstoff-Gas.

Die Umsetzung kann im batch-Verfahren oder kontinuierlich durchgeführt werden. Stets sollte in der Reaktionsmischung mindestens die stöchiometrisch zum Halogen-Fluoraustausch bzw. zur Fluorwasserstoffaddition notwendige Menge an Fluorwasserstoff vorhanden sein.

Die Aufarbeitung kann durch Leiten der Reaktionsprodukte durch einen Gaswäscher und anschließende fraktionierte Destillation erfolgen.

Die für die Durchführung verwendete Apparatur sollte gegenüber Fluorwasserstoff resistent sein. Zweckmäßig verwendet man Bauteile aus Teflon und speziellen Legierungen wie "Hastelloy", einer fluorwasserstoffresistenten Nickellegierung.

Ein weiterer Gegenstand der Erfindung ist die Verwendung von Metall-Fluorsulfonat-Verbindungen der allgemeinen Formel (IV)

$MX_n(FSO_3)_{5-n}$    (IV)

worin

    X    = Halogen

n = 0 bis 4

bedeutet, als Katalysator.

X bedeutet bevorzugt Chlor oder Fluor, und n bedeutet bevorzugt 1 bis 4, insbesondere 2 bis 4.

Die Herstellung erfolgt wie weiter oben beschrieben aus den Pentahalogeniden mit Fluorsulfonsäure oder Fluorsulfonsäurederivaten, beispielsweise Fluorsulfonsäureestern. Sofern man von Chloriden ausgeht, kann gewünschtenfalls das Reaktionsprodukt mit Fluorwasserstoff umgesetzt werden und das entsprechende Fluorid-Fluorsulfonat erhalten werden. Ausgehend von Fluoriden werden auch bei der Umsetzung mit Schwefeltrioxid entsprechende Metall-Fluorid-Fluorsulfonate erhalten.

Ein weiterer Gegenstand der Erfindung sind Zubereitungen zur Anwendung im erfindungsgemäßen Verfahren, welche eine Tantal- bzw. Niobhalogenid-Fluorsulfonat-Verbindung der Formel (IV) und entweder ein Alken der Formel (II), ein Alkan der Formel (III) oder Fluorwasserstoff, enthalten, wobei die Summe der Bestandteile 100 Gew.-% beträgt. Diese Zubereitungen, die vorzugsweise 0.1 bis 99 Gew.-%, insbesondere 10 bis 90 und ganz besonders bevorzugt 30 bis 70 Gew.-% der Verbindung der Formel (IV) enthalten, sind durch Vermischen der einzelnen Bestandteile erhältlich. Gewünschtenfalls kann etwaig gebildeter Chlorwasserstoff oder Bromwasserstoff abgetrennt werden.

Die durch das erfindungsgemäße Verfahren erhaltenen fluorhaltigen Ethanderivate sind wertvolle umweltverträgliche Lösungsmittel, Treibmittel und Zwischenprodukte für die chemische Synthese.

Das erfindungsgemäße Verfahren zeichnet sich durch hohen Umsatz und hohe Selektivität aus, und es kann vorteilhafterweise in der flüssigen Phase durchgeführt werden.

Die hohe Wirksamkeit des erfindungsgemäßen Verfahrens muß als überraschend und unvorhersehbar angesehen werden. Setzt man nämlich beispielsweise Perchlorethylen als Ausgangsverbindung und Fluorsulfonsäure als Katalysator ein, ist der Umsatz verschwindend gering. Niobpentachlorid als Katalysator liefert geringere Umsatzgrade. Es ist daher völlig unvorhersehbar, daß z. B. das Reaktionsprodukt dieser für sich allein fast nicht brauchbaren Katalysatorbestandteile im erfindungsgemäßen Verfahren zu derartig guten Ergebnissen bezüglich Umsatzgrad, Selektivität und Lebensdauer des Katalysatorgemisches führt und auch die Herstellung höher fluorierter Produkte wie insbesondere R123 gestattet.

Die folgenden Beispiele sollen das erfindungsgemäße Verfahren weiter erläutern, ohne es in seinem Umfang einzuschränken.

Beispiele

Beispiel 1:

Herstellung von Trifluordichlorethan (R123) und Difluortrichlorethan (R122) aus Tetrachlorethylen unter Verwendung einer Tantal-Halogenid-Fluorsulfonat-Verbindung

1.1. Verwendete Apparatur:

Die Umsetzung wurde in einem verschließbaren Reaktor aus HF-resistentem Material durchgeführt (Hastelloy C4™, ein legierter Stahl). Der Reaktor weist ein Innenvolumen von 2 Litern auf. Er ist mit einem Flügelrührer, Rückflußkühler, Thermostutzen und Einleitungsrohren versehen (über den Thermostutzen kann die Innentemperatur gemessen werden). Der Rückflußkühler ist mit einem mit Wasser gefüllten Gaswäscher verbunden. Der Gaswäscher seinerseits ist mit einer Tiefkühlkondensations-Einrichtung verbunden.

1.2. Herstellung der Tantal-Halogenid-Fluorsulfonat-Ver bindung

In den Reaktor wurden über Einleitungsrohre 720 g Tantalpentachlorid (2 Mol) und 230 g Fluorsulfonsäure (2,3 Mol) eingegeben. Dann wurde der Reaktor verschlossen. Die Reaktionsmischung wurde bei Raumtemperatur gerührt, wobei der Druck im Reaktor auf 5 bar anstieg. Dann wurden noch 240 g Fluorwasserstoff (12 Mol) in den Reaktor gepreßt. Der Reaktorinhalt wurde dann noch einmal bei Raumtemperatur gerührt. Anschließend wurden Bestandteile, die bei Raumtemperatur gasförmig waren, aus dem Reaktor über den Rückflußkühler abgelassen und durch den Gaswäscher geleitet. Die gasförmigen Bestandteile bestanden im wesentlichen aus HCl. Wie eine Analyse des Gaswäscherinhalts ergab, enthielt die Waschlösung den gesamten Chlor-Gehalt des eingebrachten Tantalpentachlorids in Form von Salzsäure. Es wird deshalb angenommen, daß das Tantal in der im Reaktor hergestellten Zubereitung im wesentlichen als $TaF_4$ $(FSO_3)$ in Fluorwasserstoff vorlag.

### 1.3. Durchführung der Herstellung von R123 und R122

In die nach 1.2. hergestellte Fluorwasserstoff und Tantal-Fluorid-Fluorsulfonat enthaltende Zubereitung wurden Fluorwasserstoff und Tetrachlorethylen eingeleitet. Die Dosierung wurde so eingeregelt, daß pro Stunde 50 g Fluorwasserstoff (2,5 Mol) und 50 ml Tetrachlorethylen (0,5 Mol) eindosiert wurden. Die Temperatur in der Reaktionsmischung wurde auf 145 °C, der Druck mittels eines Regelventils auf 13 bis 15 bar eingeregelt. Insgesamt wurden 4 kg Fluorwasserstoff und 4,2 kg Perchlorethylen zudosiert.

Über den Rückflußkühler wurde kontinuierlich eine adequate Menge der Leichtsieder gasförmig aus dem Reaktor abgelassen, durch den Wäscher geführt und die den Wäscher verlassenden organischen Bestandteile in der Tiefkühlkondensations-Einrichtung kondensiert. Insgesamt wurden 3.300 g organischer Bestandteile in der Tiefkühlkondensations-Einrichtung kondensiert.

Die Zusammensetzung der organischen Phase wurde gaschromatographisch bestimmt.

| Zusammensetzung: | R124 ($C_2HClF_4$) | 0,1 % |
|---|---|---|
| | R123 ($C_2HCl_2F_3$) | 38,2 % |
| | R122 ($C_2HCl_3F_2$) | 60,3 % |
| | R121 ($C_2HCl_4F$) | 0,2 % |
| | $C_2Cl_4$ | 0,9 % |
| (Angaben in Gew.-%) | | |

### Beispiel 2:

Halbkontinuierliche Herstellung von Trifluordichlorethan

#### 2.1. Verwendete Apparatur

Verwendet wurde ein Laborautoklav aus V4A-Stahl (ein mit Chrom, Nickel und Molybdän legierter Stahl). Das Innenvolumen dieses Autoklaven beträgt 0,25 Liter. Der Autoklav ist mit einem Magnetrührer, einem Tauchrohr, über welches die Ausgangsverbindungen eindosiert werden können, und einem Thermostutzen, über welchen die Innentemperatur gemessen werden kann, ausgerüstet. Der Laborautoklav weist weiterhin einen Gasauslaß auf, der mit einem mit Wasser gefüllten Gaswäscher verbunden ist. Der Gaswäscher seinerseits ist mit einer Tiefkältekondensations-Einrichtung verbunden.

#### 2.2. Herstellung der Niobhalogenid-Fluorsulfonat-Verbindung und Versuchsdurchführung

In den Autoklaven wurden 27 g Niobpentachlorid (0,1 Mol) und 10 g Fluorsulfonsäure eingebracht. Vor der Zugabe von 40 g Tetrachlorethylen (0,24 Mol) wurde der bei der Bildung der Niob-Chlorid-Fluorsulfonat-Verbindung sich bildende Chlorwasserstoff wurde abgelassen. Es wurden dann 40 g Fluorwasserstoff in 3 Portionen zudosiert. Zwischendurch wurde der entstehende Überdruck durch Ablassen leichtflüchtiger Bestandteile abgebaut.

Der Autoklav wurde dann verschlossen und einer ersten Aufheizphase unterworfen. Hierzu wurde der Autoklaveninhalt auf die in Tabelle 2 angegebene Temperatur gebracht und während der in Tabelle 2 angegebenen Zeitdauer bei dieser Temperatur gehalten. Anschließend wurde der Autoklaveninhalt auf Umgebungstemperatur gebracht und entstandener Chlorwasserstoff abgelassen. Anschließend wurde eine Probe der bei Umgebungstemperatur und Normaldruck flüchtigen Verbindungen entnommen und im Hinblick auf die darin enthaltenen organischen Verbindungen gaschromatographisch untersucht. Die Analysenwerte, angegeben in Gew.-%, finden sich in Tabelle 2.

Anschließend wurde der Autoklaveninhalt einer zweiten Aufheizphase unterworfen. Nach Abkühlen wurde der Reaktor auf Normaldruck gebracht und diesmal der gesamte Gehalt der bei Raumtemperatur und Normaldruck flüchtigen Verbindungen aus dem Reaktor abgelassen und durch den Gaswäscher geleitet. Das den Gaswäscher verlassende Rohprodukt wurde gaschromatographisch untersucht.

In den Reaktor, in welchem neben schwerflüchtigen organischen Verbindungen auch das Katalysatorsystem verblieben war, wurden dann erneut 40 g Tetrachlorethylen und 40 g Fluorwasserstoff eindosiert. Der Reaktor wurde dann einer dritten Aufheizphase unterworfen, abgekühlt, entstandener Chlorwasserstoff wurde abgelassen und anschließend eine Probe der bei Umgebungstemperatur und Normaldruck flüchtigen organischen Verbindungen analysiert. Daraufhin wurde der Reaktor einer vierten Aufheizphase unterworfen,

EP 0 533 003 B1

abgekühlt, und der gesamte Anteil der bei etwa Raumtemperatur und Normaldruck flüchtigen Verbindungen aus dem Reaktor in den Gaswäscher geleitet und das den Gaswäscher verlassende Rohprodukt wiederum gaschromatographisch untersucht.

Diese Vorgehensweise wurde mehrfach wiederholt. Nach der zweiten (siehe oben), vierten und sechsten Aufheizphase wurden jeweils 40 g Fluorwasserstoff und jeweils 40 g Tetrachlorethylen in den Reaktor eingebracht. Die in jeder Aufheizphase eingestellte maximale Temperatur, der erreichte maximale Druck und die Zeitdauer, während welcher die maximale Temperatur aufrechterhalten wurde, sowie die Daten der gaschromatographischen Analyse der nach jeder Aufheizphase erhaltenen Gasphase sind in Tabelle 2 zusammengestellt.

Tabelle 2

| Aufheizphase | Temp. max. | Druck max | Dauer | R22 | R23 | R122 | R123 |
|---|---|---|---|---|---|---|---|
| 1 | 160 °C | 34 bar | 3.5 h | -- | -- | 38,9 % | 58,7 % |
| 2 | 170 °C | 19 bar | 4.0 h | -- | 0,1 % | 16,7 % | 81,2 % |
| 3 | 160 °C | 17 bar | 3.0 h | 4,5 % | 2,6 % | 14,6 % | 76,0 % |
| 4 | 170 °C | 33 bar | 4.5 h | 1,5 % | 2,6 % | 18,4 % | 75,4 % |
| 5 | 170 °C | 26 bar | 5.0 h | 1,3 % | 2,8 % | 31,4 % | 62,1 % |
| 6[a)] | 165 °C | 28 bar | 5.5 h | 0,9 % | 2,4 % | 17,9 % | 59,8 % |
| 7[b)] | 160 °C | 15 bar | 6.0 h | 0,5 % | 0,6 % | 21,7 % | 56,3 % |
| 8[c)] | 165 °C | 17 bar | 5.0 h | -- | -- | -- | 69,6 % |
| "%" bedeutet "Gew.-%" | | | | | | | |

a) ferner 16,8 % R123 a
b) ferner 19,2 % R123 a
c) ferner 28,2 % R123 a

Bei der gaschromatischen Untersuchung wurden außerdem zwischen 0,2 und 0,8 % R124 und zwischen 0,2 und 0,5 % R125 gefunden.

Beispiel 3:

Halbkontinuierliche Herstellung von R123 und R122 aus Tetrachlorethylen

3.1. Verwendete Apparatur

Es wurde die unter Beipiel 2.1. beschriebene Apparatur verwendet.

3.2. Herstellung einer katalytisch aktiven Zubereitung, enthaltend eine Tantal-Halogenid-Fluorsulfonat-Verbindung

40 g Tetrachlorethylen, 36 g (0,1 mol) Tantalpentachlorid und 30 g (0,3 mol) Fluorsulfonsäure wurden in dieser Reihenfolge im Autoklaven vorgelegt (hierbei wirkte das Tetrachlorethylen wie ein Verdünnungsmittel). Der Autoklav wurde verschlossen und der Inhalt des Autoklaven gerührt. Während des Rührens baute sich ein Druck von etwa 5 bar auf. Dieser Druck wurde über das Regelventil abgelassen. Es lag dann eine Zubereitung vor, die die gebildete Tantal-Chlorid-Fluorsulfonat-Verbindung und Tetrachlorethylen enthielt.

3.3. Versuchsdurchführung

In die in Beispiel 3.2. hergestellte Zubereitung wurden 40 g Fluorwasserstoff dosiert. Der Druck stieg beim Rühren auf etwa 12 bar an. Der Druck wurde über das Regelventil abgelassen.

In einer ersten Aufheizphase wurde der Reaktorinhalt binnen einer Stunde auf 160 °C aufgeheizt und 4 Stunden bei dieser Temperatur gehalten. Der Druck stieg dabei bis auf 32 bar. Nach dem Abkühlen wurden die Leichtsieder gaschromatographisch untersucht. Das Analysenergebnis ist in Tabelle 3 zusammengestellt.

Vor einer zweiten Aufheizphase wurden 20 g Tetrachlorethylen und 20 g Fluorwasserstoff in den Autoklaven eingebracht. In analoger Weise wurden insgesamt 8 Aufheizphasen durchgeführt. Zwischen den Auf-

EP 0 533 003 B1

heizphasen wurden die Leichtsieder gaschromatographisch untersucht (das Untersuchungsergebnis der siebten Aufheizphase war verfälscht) und jeweils 20 g Tetrachlorethylen und 20 g Fluorwasserstoff in den Autoklaven eingebracht (vor der sechsten Aufheizphase nur 20 g Fluorwasserstoff, aber kein Tetrachlorethylen). Insgesamt wurden 180 g Tetrachlorethylen und 200 g Fluorwasserstoff eingesetzt. Die Verfahrensparameter und das Analysenergebnis der einzelnen Aufheizphasen (bis auf die siebte Aufheizphase) sind in Tabelle 3 zusammengestellt.

Nach Beendigung der letzten Aufheizphase wurde die im Autoklaven verbliebene Reaktionsmischung auf Eis gegossen und 74 g einer sich abscheidenden organischen Phase abgetrennt. Auch diese organische Phase wurde gaschromatographisch untersucht. 40 g des eingesetzten Tetrachlorethylens wurden so zurückgewonnen.

Aus den Analyseergebnissen konnte berechnet werden, daß die insgesamt 140 g Tetrachlorethylen, die im Verlauf des halbkontinuierlichen Verfahrens umgesetzt worden waren, im wesentlichen zu R123 (70 %), R122 (25 %), R121 (5 %) umgewandelt wurden.

Tabelle 3

| Aufheiz phase | Temp. max. | Druck max | Dauer | R124 | R133a | R122 | R123 |
|---|---|---|---|---|---|---|---|
| 1 | 160 °C | 32 Bar | 6 h | 4,1 % | 1,7 % | 1,4 % | 91,4 % |
| 2 | 160 °C | 22 Bar | 3 h | 1 % | 4 % | 4,5 % | 90 % |
| 3 | 164 °C | 23 Bar | 4,5 h | 0,6 % | 0,6 % | 29,3 % | 67,8 % |
| 4 | 163 °C | 18 Bar | 3,5 h | 0,4 % | 1,1 % | 16,8 % | 79,7 % |
| 5 | 160 °C | 16 Bar | 2 h | 0,4 % | 1,4 % | 12,7 % | 82,6 % |
| 6[a] | 160 °C | 16 Bar | 4,5 h | --- % | --- % | --- % | --- % |
| 7[b] | 160 °C | 16 Bar | 3,5 h | 0,4 % | --- % | 2,3 % | 64,3 % |
| 8[c] | 160 °C | 15 Bar | 6 h | 1,2 % | 0,3 % | 26,4 % | 65,6 % |
| Rückstand | ---- | ---- | --- | --- | --- | 29,8 % | [d] 0.4 % |
| "%" bedeutet "Gew.-%" | | | | | | | |

a) GC-Probe verfälscht
b) ferner 31 % $R23/CO_2$, 1,1 % R22, 0,3 % R1112
c) ferner 4,5 % R1112, 0,3 % R113
d) ferner 41,6 % Tetrachlorethylen, 27,6 % R 121

Der Anteil höherfluorierter Ethane, insbesondere Trifluordichlorethan sowie Difluortrichlorethan ist auch bei kontinuierlicher oder halbkontinuierlicher Verfahrensweise aufgrund der Standfestigkeit des Katalysators auch nach langer Reaktionsdauer noch sehr gut.

**Patentansprüche**

1. Verfahren zur Herstellung von Fluor enthaltenden Ethanderivaten der allgemeinen Formel (I)

$$F_kH_nCl_{3-k-n}C\text{-}CZ^1Z^2F \qquad (I)$$

worin $Z^1$ und $Z^2$ gleich oder verschieden sein können und Wasserstoff, Fluor, Chlor oder Brom bedeuten, k 0, 1 oder 2 und n 1, 2 oder 3 bedeuten

durch Umsetzung eines Alkens oder halogenhaltigen Alkans als Ausgangsverbindung mit Fluorwasserstoff in flüssiger Phase bei einer Temperatur zwischen 0 und 250 °C, wobei die Umsetzung durch eine katalytisch aktive Zubereitung enthaltend eine Tantal-Fluorsulfonat-Verbindung und/oder eine Niob-Fluorsulfonat-Verbindung, die im wesentlichen frei von Tantal- und/oder Niobpentahalogenid, vorzugsweise im wesentlichen frei von Tantal- und/oder Niobpentachlorid und Tantal- und/oder Niobpentabromid ist, katalysiert wird und der Fluorwasserstoff mindestens in der äquimolaren Menge, bezogen auf die Ausgangsverbindung, eingesetzt wird, das Mol-Verhältnis von Ausgangsverbindung zu katalytisch aktiver Verbindung zwischen etwa 10:1 bis 1:100 beträgt, wobei man
   a) als Alken eine Verbindung der allgemeinen Formel (II) verwendet

$$F_kH_mCl_{2-k-m}C = CX^1X^2 \qquad (II)$$

worin $X^1$ und $X^2$ gleich oder verschieden sein können und Wasserstoff, Fluor, Chlor oder Brom bedeuten, k die oben angegebene Bedeutung besitzt und m 0, 1 oder 2 bedeutet, oder
b) man als halogenhaltiges Alkan eine Verbindung der allgemeinen Formel (III) verwendet

$$F_kH_nCl_{3-k-n}C\text{-}CY^1Y^2Y^3 \qquad (III)$$

worin k und n die obige Bedeutung besitzen, $Y^1$, $Y^2$ gleich oder verschieden sein können und Wasserstoff, Fluor, Chlor oder Brom und $Y^3$ Chlor oder Brom bedeuten.

2. Verfahren nach Anspruch 1, dadurch gekennzeichnet, daß man bei einer Temperatur zwischen etwa 50 und 250 °C und einem Druck von 1 bis 100 bar (abs.) arbeitet.

3. Verfahren nach Anspruch 2, dadurch gekennzeichnet, daß man zur Herstellung von Fluor enthaltenden Ethanderivaten der allgemeinen Formel (Ia)

$$F_kH_nCl_{3-k-n}C\text{-}CF_3 \qquad (Ia)$$

worin k 0, 1 oder 2 und n 1, 2 oder 3 bedeutet, bei einer Temperatur von 50 bis 250 °C und einem Druck von 1 bis 100 bar
a) ein halogenhaltiges Alken der allgemeinen Formel

$$F_kH_mCl_{2-k-m}C = CX^1X^2 \qquad (II),$$

worin k und m die oben angegebene Bedeutung besitzen, $X^1$ und $X^2$ Fluor, Chlor oder Brom bedeuten, mit Fluorwasserstoff umsetzt, wobei der Fluorwasserstoff mindestens in der stöchiometrisch zur Fluorwasserstoffanlagerung und zum Halogen-Fluor-Austausch beim Alken notwendigen Menge eingesetzt wird oder
b) ein halogenhaltiges Alkan der allgemeinen Formel

$$F_kH_nCl_{3-k-n}C\text{-}CY^1Y^2Y^3 \qquad (III)$$

worin k und n die oben angegebene Bedeutung besitzen, $Y^1$, $Y^2$ Fluor, Chlor oder Brom und $Y^3$ Chlor oder Brom bedeuten, mit Fluorwasserstoff umsetzt, wobei der Fluorwasserstoff mindestens in der stöchiometrisch zum Halogen- Fluor-Austausch beim Alkan notwendigen Menge eingesetzt wird.

4. Verfahren nach Anspruch 3, dadurch gekennzeichnet, daß man zur Herstellung von $CHCl_2CF_3$
a) $CCl_2 = CCl_2$ mit Fluorwasserstoff umsetzt, wobei Fluorwasserstoff mindestens in der stöchiometrisch zur Fluorwasserstoff-Anlagerung und zum Chlor-Fluor Austausch am Alken notwendigen Menge eingesetzt wird, oder
b) $CHCl_2CCl_3$, $CHCl_2CFCl_2$, $CHCl_2CF_2Cl$ oder deren Gemische mit Fluorwasserstoff umsetzt, wobei Fluorwasserstoff mindestens in der stöchiometrisch zum Chlor-Fluor-Austausch am Alkan notwendigen Menge eingesetzt wird.

5. Verfahren nach einem der vorhergehenden Ansprüche, dadurch gekennzeichnet, daß man von einem halogenierten Alken ausgeht.

6. Verfahren nach Anspruch 4 oder 5, dadurch gekennzeichnet, daß man zur Herstellung von $CHCl_2CF_3$ ausgeht von $CCl_2 = CCl_2$.

7. Verfahren nach Anspruch 6, dadurch gekennzeichnet, daß das Mol-Verhältnis von $CCl_2 = CCl_2$ und Fluorwasserstoff zwischen 1:3 und 1:100 liegt.

8. Verfahren nach Anspruch 3, dadurch gekennzeichnet, daß man bei einer Temperatur von 70 bis 220 °C und einem Druck von 10 bis 50 bar (abs.) arbeitet.

9. Verfahren nach einem der vorhergehenden Ansprüche, dadurch gekennzeichnet, daß die katalytisch aktive Verbindung eine Tantal-Halogenid-Fluorsulfonat-Verbindung oder eine Niob-Halogenid-Fluorsulfonat-Verbindung ist.

**10.** Verfahren nach Anspruch 9, dadurch gekennzeichnet, daß die katalytisch aktive Verbindung eine TantalChlorid-Fluorsulfonat-Verbindung oder eine Niob-Chloid-Fluorsulfonat-Verbindung ist, die durch Umsetzung von Tantalpentachlorid oder Niobpentachlorid mit Fluorsulfonsäure oder Fluorsulfonsäurederivaten, die mit Tantalpentachlorid oder Niobpentachlorid unter Substitution von Chlorid durch Fluorsulfonat reagieren, im Molverhältnis von 1:1 bis 1:4 erhalten wurde.

**11.** Verfahren nach Anspruch 9, dadurch gekennzeichnet, daß die katalytisch aktive Verbindung eine Tantal-Fluorid-Fluorsulfonat-Verbindung oder eine Niob-Fluorid-Fluorsulfonat-Verbindung ist, die durch Umsetzung von Tantalpentafluorid oder Niobpentafluorid mit Schwefeltrioxid oder durch Umsetzung von Tantalpentachlorid oder Niobpentachlorid mit Fluorsulfonsäure oder einem Fluorsulfonsäurederivat, welches mit Tantalpentachlorid oder Niobpentachlorid unter Substitution von Chlorid durch Fluorsulfonat reagiert, im Molverhältnis von 1:1 bis 1:4 und anschließendem Chlor-Fluor-Austausch erhalten wurde.

**12.** Verfahren nach einem der vorhergehenden Ansprüche, dadurch gekennzeichnet, daß das Mol-Verhältnis von Ausgangsverbindung zum Katalysatorgemisch zwischen etwa 10:1 und 1:10 liegt.

**13.** Verwendung von Metall-Fluorsulfonat-Verbindungen der allgemeinen Formel (IV)

$$MX_n(FSO_3)_{5-n} \qquad (IV)$$

worin
$X \qquad = Hal$
$M \qquad = Tantal, Niob$
$n \qquad = 0 \text{ bis } 4$
bedeutet, als Katalysator.

**14.** Verwendung nach Anspruch 13, dadurch gekennzeichnet, daß
$X \qquad = Chlor, Fluor,$
$n \qquad = 2 \text{ bis } 4$
bedeutet.

**15.** Zubereitung zur Anwendung in einem Verfahren gemäß den Ansprüchen 1 bis 12, enthaltend eine Tantal-Halogenid-Fluorsulfonat-Verbindung oder eine Niob-Halogenid-Fluorsulfonat-Verbindung der Formel (IV) und entweder ein Alken der Formel (II), ein Alkan der Formel (III) oder Fluorwasserstoff.

**Claims**

**1.** Process for the preparation of fluorine-containing ethane derivatives of the general formula (I)

$$F_kH_nCl_{3-k-n}C\text{-}CZ^1Z^2F \qquad (I)$$

wherein $Z^1$ and $Z^2$ can be identical or different and denote hydrogen, fluorine, chlorine or bromine, k denotes 0, 1 or 2 and n denotes 1, 2 or 3,
by reaction of an alkene or halogen-containing alkane, as the starting compound, with hydrogen fluoride in a liquid phase at a temperature of between 0 and 250 °C, the reaction being catalysed by a catalytically active formulation containing a tantalum fluorosulphonate compound and/or a niobium fluorosulphonate compound which is essentially free from tantalum pentahalide and/or niobium pentahalide, preferably essentially free from tantalum pentachloride and/or niobium pentachloride and tantalum pentabromide and/or niobium pentabromide, and the hydrogen fluoride being used at least in the equimolar amount, based on the starting compound, the molar ratio of starting compound to catalytically active compound being between about 10:1 and 1:100, wherein
a) a compound of the general formula (II)

$$F_kH_mCl_{2-k-m}C = CX^1X^2 \qquad (II)$$

wherein $X^1$ and $X^2$ can be identical or different and denote hydrogen, fluorine, chlorine or bromine, k has the abovementioned meaning and m denotes 0, 1 or 2, is used as the alkene, or
b) a compound of the general formula (III)

$$F_kH_nCl_{3-k-n}C\text{-}CY^1Y^2Y^3 \qquad (III)$$

wherein k and n have the above meaning, $Y^1$ and $Y^2$ can be identical or different and denote hydrogen, fluorine, chlorine or bromine and $Y^3$ denotes chlorine or bromine, is used as the halogen-containing alkane.

2. Process according to Claim 1, characterised in that it is carried out at a temperature between about 50 and 250°C and under a pressure of 1 to 100 bar (absolute).

3. Process according to Claim 2, characterised in that, to prepare fluorine-containing ethane derivatives of the general formula (Ia)

$$F_kH_nCl_{3-k-n}C\text{-}CF_3 \qquad (Ia)$$

wherein k denotes 0, 1 or 2 and n denotes 1, 2 or 3, at a temperature of 50 to 250°C and under a pressure of 1 to 100 bar,
a) a halogen-containing alkene of the general formula

$$F_kH_mCl_{2-k-m}C = CX^1X^2 \qquad (II)$$

wherein k and m have the abovementioned meaning and $X^1$ and $X^2$ denote fluorine, chlorine or bromine, is reacted with hydrogen fluoride, the hydrogen fluoride being employed in at least the stoichiometrically required amount for adding on the hydrogen fluoride and for the halogen-fluorine exchange on the alkene, or
b) a halogen-containing alkane of the general formula

$$F_kH_nCl_{3-k-n}C\text{-}CY^1Y^2Y^3 \qquad (III)$$

wherein k and n have the abovementioned meaning, $Y^1$ and $Y^2$ denote fluorine, chlorine or bromine and $Y^3$ denotes chlorine or bromine, is reacted with hydrogen fluoride, the hydrogen fluoride being employed in at least the stoichiometrically required amount for the halogen/fluorine exchange on the alkane.

4. Process according to Claim 3, characterised in that, to prepare $CHCl_2CF_3$,
a) $CCl_2 = CCl_2$ is reacted with hydrogen fluoride, the hydrogen fluoride being employed in at least the stoichiometrically required amount for adding on hydrogen fluoride and for the chlorine/fluorine exchange on the alkene, or
b) $CHCl_2CCl_3$, $CHCl_2CFCl_2$, $CHCl_2CF_2Cl$ or mixtures thereof are reacted with hydrogen fluoride, the hydrogen fluoride being employed in at least the stoichiometrically required amount for the chlorine/fluorine exchange on the alkane.

5. Process according to one of the preceding claims, characterised in that a halogenated alkene is used as the starting substance.

6. Process according to Claim 4 or 5, characterised in that $CCl_2 = CCl_2$ is used as the starting substance for the preparation of $CHCl_2CF_3$.

7. Process according to Claim 6, characterised in that the molar ratio of $CCl_2 = CCl_2$ and hydrogen fluoride is between 1:3 and 1:100.

8. Process according to Claim 3, characterised in that it is carried out at a temperature of 70 to 220°C under a pressure of 10 to 50 bar (absolute).

9. Process according to one of the preceding claims, characterised in that the catalytically active compound is a tantalum halide fluorosulphonate compound or a niobium halide fluorosulphonate compound.

10. Process according to Claim 9, characterised in that the catalytically active compound is a tantalum chloride fluorosulphonate compound or a niobium chloride fluorosulphonate compound which has been obtained by reaction of tantalum pentachloride or niobium pentachloride with fluorosulphonic acid or fluorosulphonic acid derivatives which react with tantalum pentachloride or niobium pentachloride, chloride being replaced by fluorosulphonate, in a molar ratio of 1:1 to 1:4.

11. Process according to Claim 9, characterised in that the catalytically active compound is a tantalum fluoride fluorosulphonate compound or a niobium fluoride fluorosulphonate compound which has been obtained by reaction of tantalum pentafluoride or niobium pentafluoride with sulphur trioxide or by reaction of tantalum pentachloride or niobium pentachloride with fluorosulphonic acid or a fluorosulphonic acid derivative which reacts with tantalum pentachloride or niobium pentachloride, chloride being rePlaced by fluorosulphonate, in a molar ratio of 1:1 to 1:4 and subsequent chlorine/fluorine exchange.

12. Process according to one of the preceding claims, characterised in that the molar ratio of starting compound to catalyst mixture is between about 10:1 and 1:10.

13. The use of metal fluorosulphonate compounds of the general formula (IV)

$$MX_n(FSO_3)_{5-n} \qquad (IV)$$

wherein
X denotes Hal,
M denotes tantalum or niobium and
n denotes 0 to 4
as a catalyst.

14. The use according to Claim 13, characterised in that
X denotes chlorine or fluorine and
n denotes 2 to 4.

15. Formulation for use in a process according to Claims 1 to 12, containing a tantalum halide fluorosulphonate compound or a niobium halide fluorosulphonate compound of the formula (IV), and either an alkene of the formula (II), an alkane of the formula (III) or hydrogen fluoride.

**Revendications**

1. Procédé de préparation de dérivés de l'éthane contenant du fluor, de formule générale (I)
$$F_kH_nCl_{3-k-n}C-CZ^1Z^2F \qquad (I)$$

dans laquelle $Z^1$ et $Z^2$ peuvent être identiques ou différents, et représentent l'hydrogène, le fluor, le chlore ou le brome, k vaut 0, 1 ou 2, et n vaut 1, 2 ou 3,
par réaction d'un alcène ou d'un alcane halogéné, servant de composé de départ, avec de l'acide fluorhydrique en phase liquide, à une température comprise entre 0 et 250°C, la réaction étant catalysée par une préparation à activité catalytique contenant un composé de type fluorosulfonate de tantale et/ou un composé de type fluorosulfonate de niobium, qui pour l'essentiel est exempt de pentahalogénure de tantale et/ou de niobium, et de préférence est essentiellement exempt de pentachlorure de tantale et/ou de niobium et de pentabromure de tantale et/ou de niobium, et que l'acide fluorhydrique est utilisé au moins en une quantité équimolaire par rapport au composé de départ, le rapport en moles du composé de départ au composé à activité catalytique étant compris entre environ 10:1 et 1:100, procédé dans lequel :
a) on utilise comme alcène un composé de formule générale (II)

$$F_kH_mCl_{2-k-m}C = CX^1X^2 \qquad (II)$$

dans laquelle $X^1$ et $X^2$ peuvent être identiques ou différents et représentent chacun un hydrogène,

14

un fluor, un chlore ou un brome, k a les significations données ci-dessus, et m vaut 0, 1 ou 2, ou bien

b) on utilise comme alcane halogéné un composé de formule générale (III)

$$F_kH_nCl_{3-k-n}C\text{-}CY^1Y^2Y^3 \qquad (III)$$

dans laquelle k et n ont les significations ci-dessus, $Y^1$ et $Y^2$ peuvent être identiques ou différents et représentent chacun un hydrogène, un fluor, un chlore ou un brome, et $Y^3$ est le chlore ou le brome.

2. Procédé selon la revendication 1, caractérisé en ce qu'on travaille à une température comprise entre environ 50 et 250°C, sous une pression absolue de 1 à 100 bar.

3. Procédé selon la revendication 2, caractérisé en ce que, pour préparer des dérivés de l'éthane contenant du fluor de formule générale (Ia)

$$F_kH_nCl_{3-k-n}C\text{-}CF_3 \qquad (Ia)$$

dans laquelle k vaut 0, 1 ou 2 et n vaut 1, 2 ou 3, et en procédant à une température de 50 à 250°C et sous une pression de 1 à 100 bar

a) on fait réagir avec de l'acide fluorhydrique un alcène halogéné de formule générale

$$F_kH_mCl_{2-k-m}C = CX^1X^2 \qquad (II)$$

dans laquelle k et m ont les significations données ci-dessus, $X^1$ et $X^2$ représentent le fluor, le chlore ou le brome, auquel cas l'acide fluorhydrique est utilisé en quantité stoechiométriquement nécessaire à l'addition de l'acide fluorhydrique et à l'échange halogène-fluor dans l'alcène, ou bien

b) on fait réagir avec de l'acide fluorhydrique un alcane halogéné de formule générale

$$F_kH_nCl_{3-k-n}C\text{-}CY^1Y^2Y^3 \qquad (III)$$

dans laquelle k et n ont les significations données ci-dessus, $Y^1$ et $Y^2$ sont chacun le fluor, le chlore ou le brome, et $Y^3$ est le chlore ou le brome, où l'acide fluorhydrique est utilisé au moins en la quantité stoechiométriquement nécessaire à l'échange halogène-fluor dans l'alcane.

4. Procédé selon la revendication 3, caractérisé en ce que, pour la préparation du $CHCl_2CF_3$,
   a) on fait réagir du $CCl_2 = CCl_2$ avec de l'acide fluorhydrique, en utilisant l'acide fluorhydrique au moins en la quantité stoechiométriquement nécessaire à l'addition de l'acide fluorhydrique et à l'échange chlore-fluor dans l'alcène, ou bien
   b) on fait réagir avec de l'acide fluorhydrique du $CHCl_2CCl_3$, du $CHCl_2CFCl_2$, du $CHCl_2CF_2Cl$ ou leurs mélanges, auquel cas on utilise l'acide fluorhydrique au moins en la quantité stoechiométriquement nécessaire à l'échange chlore-fluor dans l'alcane.

5. Procédé selon l'une des revendications précédentes, caractérisé en ce qu'on part d'un alcène halogéné.

6. Procédé selon la revendication 4 ou 5, caractérisé en ce que, pour préparer le $CHCl_2CF_3$, ou part de $CCl_2 = CCl_2$.

7. Procédé selon la revendication 6, caractérisé en ce que le rapport en moles du $CCl_2 = CCl_2$ à l'acide fluorhydrique est compris entre 1:3 et 1:100.

8. Procédé selon la revendication 3, caractérisé en ce qu'on travaille à une température de 70 à 220°C et sous une pression absolue de 10 à 50 bar.

9. Procédé selon l'une des revendications précédentes, caractérisé en ce que le composé à activité catalytique est un composé de type halogénure-fluorosulfonate de tantale ou halogénure-fluorosulfonate de niobium.

**10.** Procédé selon la revendication 9, caractérisé en ce que le composé à activité catalytique est un composé de type chlorure-fluorosulfonate de tantale ou un composé chlorure-fluorosulfonate de niobium, que l'on a obtenu par réaction du pentachlorure de tantale ou du pentachlorure de niobium avec l'acide fluorosulfonique ou des dérivés de l'acide fluorosulfonique, qui réagissent avec le pentachlorure de tantale ou le pentachlorure de niobium avec substitution du chlorure par le fluorosulfonate, selon un rapport en moles de 1:1 à 1:4.

**11.** Procédé selon la revendication 9, caractérisé en ce que le composé à activité catalytique est un composé de type fluorure-fluorosulfonate de tantale ou un composé de type fluorure-fluorosulfonate de niobium, que l'on a obtenu par réaction du pentafluorure de tantale ou du pentafluorure de niobium avec du trioxyde de soufre, ou par réaction du pentachlorure de tantale ou du pentachlorure de niobium avec l'acide fluorosulfonique ou un dérivé de l'acide fluorosulfonique, qui réagit avec le pentachlorure de tantale ou le pentachlorure de niobium avec substitution du chlorure par le fluorosulfonate, selon un rapport de moles de 1:1 à 1:4, l'opération étant suivie d'un échange chlore-fluor.

**12.** Procédé selon l'une des revendications précédentes, caractérisé en ce que le rapport en moles du composé de départ au mélange de catalyseurs est compris entre environ 10:1 et 1:10.

**13.** Utilisation, comme catalyseur, de composés de type fluorosulfonate métallique de formule générale (IV)

$$MX_n(FSO_3)_{5-n} \qquad (IV)$$

dans laquelle X est hal, M est le tantale ou le niobium, et n vaut de 0 à 4.

**14.** Utilisation selon la revendication 13, caractérisée en ce que X est le chlore ou le fluor, et n vaut de 2 à 4.

**15.** Préparation destinée à une utilisation dans un procédé selon les revendications 1 à 12, contenant un composé de type halogénure-fluorosulfonate de tantale ou un composé de type halogénure-fluorosulfonate de niobium de formule (IV), et un alcène de formule (II), un alcane de formule (III) ou de l'acide fluorhydrique.